Europäisches Patentamt

European Patent Office

Office européen des brevets

(11) Publication number: **0 306 569**
**A1**

## (12) EUROPEAN PATENT APPLICATION

(21) Application number: 87307906.5

(22) Date of filing: 08.09.87

(51) Int. Cl.⁴: **A61N 5/06 , A61N 1/32 ,
A61N 1/42**

(43) Date of publication of application:
**15.03.89 Bulletin 89/11**

(84) Designated Contracting States:
**AT BE CH DE ES FR GB GR IT LI LU NL SE**

(71) Applicant: **C K Chao and Company Limited
15th Floor Tai Po Commercial Centre 152-172
Kwong Fuk Road
Tai Po N T(HK)**

(72) Inventor: **Wei, Xiu-Bing
15th Floor Sanwa Building
30-32 Connaught Road Central(HK)**

(74) Representative: **Pacitti, Pierpaolo Alfonso M.E.
et al
Ian G. Murgitroyd and Company Mitchell
House 333 Bath Street
Glasgow G2 4ER(GB)**

(54) Method of and apparatus for laser treatment to stop smoking.

(57) A method of and apparatus for treatment to stop smoking comprising the steps of (a) applying a bioelectrical-stimulator (16) to stimulate two pairs of target points on the body of a subject via surface electrodes (20), (b) applying laser (14) bilaterally to a first pair (37) of auricular points, (c) applying laser (14) bilaterally to a second pair (41) of auricular points, (d) applying laser (14) bilaterally to a third pair (52) of auricular points; (e) applying magnetic discs to the three pairs of auricular points; and (f) repeating steps (a)-(e) to effect gradual reduction of smoking until the point of total abstinence is reached.

FIG. 1

EP 0 306 569 A1

## METHOD OF AND APPARATUS FOR LASER TREATMENT TO STOP SMOKING

This invention relates to a method of and apparatus for laser treatment to stop smoking, and in particular to laser treatment associating and in combination with electrotherapeutic and magnetotherapeutic treatments.

Numerous methods of treatment to stop smoking are known.

Hypnosis is one method adopted by some people to overcome smoking habit. However, it is appreciated that the attitude towards giving up smoking is a major factor to concern and therefore it is unlikely that a patient will successfully give up smoking under hypnosis unless the patient really want to do so.

Acupuncture is another method used to help patient in giving up cigarette smoking. A major disadvantage of the method is the unpleasant insertion of needles into the skin and the distention and soreness experienced. Also, acupuncture does not work if the patient undergoing acupuncture treatment does not have the will-power to quit smoking. In fact, acupuncture treatment has a higher failure rate than hypnosis.

Cigarette substitutes such as herbal cigarettes and non-tobacco cigarettes are used. Although these cigarette substitutes have no nicotine, they still produce tar and carbon monoxide when burnt and hence are harzardous in terms of cancer and heart disease.

Chewing tobacco and stuff can also serve as smoking alternatives. However, there exists the risk of leukoplakia (an occasionally precancerous condition) and oral cancer.

Nicotine chewing gum and nicotine tablet are some stop smoking nicotine alternatives. Unfortunately, these alternatives have little effect in helping patients giving up cigarette smoking and are unable to replace cigarette. Besides, there is the risk of over-consumption. Patients may consider these chewing gum and tablet as freely available form of nicotine and hence have the tendency to take more doses of nicotine through the chewing gum and tablet than through cigarettes.

It is an object of the present invention to produce a method of and apparatus for laser treatment to stop smoking that eliminates the problems experienced in the aforementioned methods.

It is another object of the present invention to provide a method of and apparatus for laser treatment to stop smoking whereby patient is allowed to taper off cigarette consumption for a limited time so that withdrawal symptoms can be mitigated.

It is a further object of the present invention to provide a method of and apparatus for laser treatment to stop smoking which has no significant physical and mental side-effects.

It is yet another object of the present invention to produce a method of and apparatus for laser treatment to stop smoking which is safe and effective.

In accordance with one aspect the invention provides a method of and apparatus for laser treatment to quit smoking comprises the steps of:-

(a) applying a bioelectrical-stimulator to the body of a subject via surface electrodes;

(b) applying lasers bilaterally to irradiate a first pair of auricular points on the auricles of the subject;

(c) applying lasers bilaterally to irradiate a second pair of auricular points on the auricles of the subject;

(d) applying lasers bilaterally to irradiate a third pair of auricular points on the auricles of the subject;

(e) applying small magnetic discs to said first, second and third pair of auricular points; and

(f) repeating the above steps to effect gradual reduction of cigarette consumption until the subject totally stop smoking.

The invention is described further, by way of illustration, with reference to the accompanying drawings, wherein:-

Figure 1 is a schematic diagram of the system of laser treatment in accordance with the present invention.

Figure 2 is an illustrative diagram showing the location of the three auricular points on a left auricle.

Figure 3 is an illustrative diagram showing the location of HEART 7 and SPLEEN 6 on a body.

Figure 4 is a block diagram showing the procedures of the treatment in accordance with the present invention.

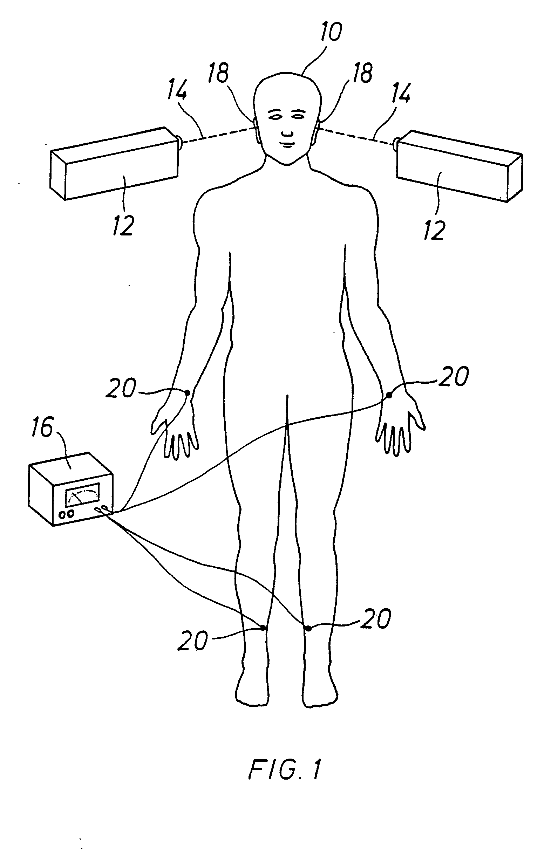

Referring now in more detail to the drawings, Figure 1 is a diagrammatic drawing of the system of the treatment according to the invention. Patient 10 is being supported on a treatment table in a clinic with his or her head being steadily positioned by a pillow or cushion.

Laser apparatus 12, 12 are positioned on both sides of the patient 10 near the head portion thereof and are adapted to be operable to produce output beams 14, 14 (represented by phantom lines) of an adjustable level.

A bioelectrical-stimulator 16 is used to produce electrical stimulation to specific target spots or points on the body of the patient 10. These target spots or points to be stimulated will be described later in detail.

The laser apparatus 12, 12 are adjusted and positioned bilaterally with respect to and at a distance from the head of the patient 10. This distance is most preferably in the region of 30 to 35 cm. Laser beams 14, 14 are directed to the respective auricles 18, 18 of the patient 10 at selected auricular points at a substantially perpendicualr angle with respect to the surface of the auricles on which the auricular points are located.

The laser output level set in the treatment is most preferably five milliwatts(mw).

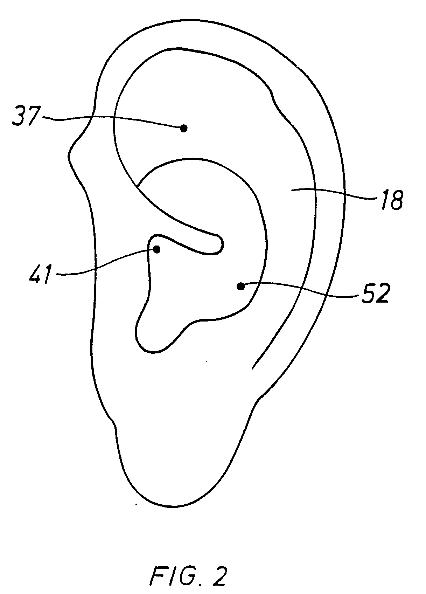

Three bilateral pairs of auricular points located on the auricles 18, 18 of the patient 10 are specially designated in the laser treatment. These three pairs of auricular points commonly known as Spirit Door, Esophagus and Lung are represented by reference numerals 37, 41 and 52 respectively as shown in Figure 2. These auricular points 37, 41 and 52 may be exactly located with the aid of an electrical acupuncture point detector.

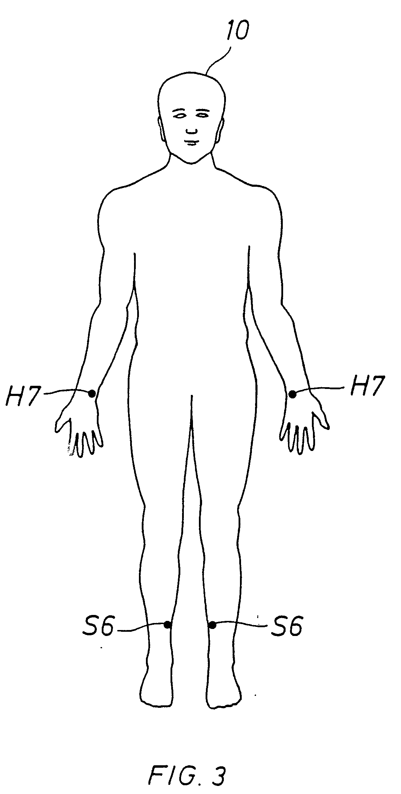

The treatment is commenced by first applying electrical stimulation to HEART 7 (H7) and SPLEEN 6 (S6) on the body of the patient 10 by means of a bioelectrical-stimulator 14. The location of HEART 7 (H7) and SPLEEN 6 (S6) on the body is shown in Figure 3. Stimulation is achieved through the use of surface electrodes 20 which are adapted for operative electrical contact with the body of the patient 10.

The surface electrodes 20 may be fabricated from metal or preferably fabricated from a conductive plastic material so as to give the patient 10 a warm and comfortable feeling.

The main purpose of the electrotherapy treatment is to drive the patient 10 to a deep state of relaxation during the laser treatment. This deep state of relaxation that is reached would help resolve many of the conditoons that are associated with stress and emotional disturbance.

The electrotherapy treatment is to be performed simultaneously with the laser treatment which will now be described.

Auricular points 37, 37 located on the auricles 18, 18 are first irradiated by the lasers 12, 12. This laser irradiation is done for at least five minutes. Thereafter, the laser beams 14, 14 are directed to the auricular points 41, 41. These auricular points 41, 41 are then irradiated for at least five minutes. Finally, the auricular points 52, 52 are irradiated for again at least five minutes.

After completing the laser and electrotherapeutic treatments, magnetotherapeutic treatment is performed. This is done by placing tiny magnetic discs (not shown) or the like at the irradiated auricular points 37, 41 and 52 on the auricles 18, 18 of the patient 10. The magnetic discs have magnetic strength of 2500 GAUS.

It is appreciated that the magnetotherapy may be carried out at the patient's own home.

The complete treatment may be administrated every day or every other day for approximately five treatments. After each treatment, the patient should reduce his or her quantity of cigarette consumption by at least one half. This gradual reduction of smoking as the treatment progresses would mitigate the withdrawal symptoms which are suffered by patients who are heavy smokers when they start to quit smoking. These withdrawal symptoms are caused by the body's adjustment to the withdrawal of a drug which it has become dependent.

The duration of the treatment varies in accordance with the patient's initial quantity of consumption as well as the rate of tapering off. For example, if the patient initially smokes 80 cigarettes a day and cuts down smoking to one half after each treatment, then he or she would be smoking 40 cigarettes per day after the first treatment. After the second treatment, the cigarette consumption is cut down to 20 cigarettes, and so on until the cigarette consumption tapered to 5 after the fourth treatment. The patient would eventually quit smoking altogether after the fifth or the last treatment.

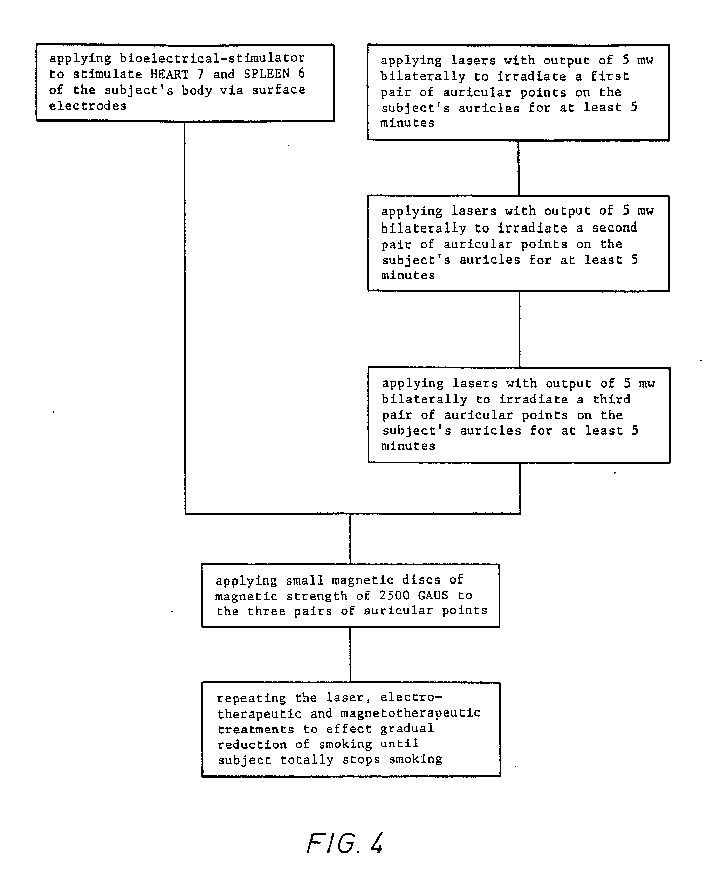

Figure 4 is a block diagram showing the procedures of the treatment described hereinbefore.

After long and extensive clinical researches made by the author, it is unveiled that laser beam having output of 5 mw produces distinct biochemical effects in a human body when irradiated on certain auricular points on the auricles. This irradiation of the laser is particularly effective in activating and enhancing the pain modulatory system of the body.

The pain modulatory system generally consists of certain organizations of the higher order central nervous system such as the caudatus nucleus, the amygdalae nucleus as well as certain pain modulatory substances. The essential substance among these pain modulatory substances is endorphin or endorphin-like substance. The increasing amount of endorphin or endorphin-like substance reduces the irritability of the receptors and hence produces quieting and pain suppressing effects.

It is understood that nicotine and certain morphine-like toxic substances identified in cigarette smoke have sedative and quieting effect upon the central nervous system. This sedative and quieting effect creates the illusion of well-being and release from tension which becomes the main reason why people are addicted to smoking.

Laser beam of specific wave length and power density irradiated on a certain receptor site of the body of a patient triggers the release of pain modulatory substances such as endorphin thereby enabling the patient to release his or her craving for addictive toxic substances in the smoke. Patient can be relieved of the dependency of the addictive drug produced in cigarette smoking and thus cigarette smoking. This is the main reason why patient would find cigarettes taste foul after the laser treatment.

Furthermore, laser irradiation upon the receptor sites leads to an increase of the transmission rate of neural impulses which gives rise to some noticeable changes of the internal function of a human body.

According to the author's research documentations, these changes in the body include activation of the activity of enzymes, increase of the amount of phagocyte, red blood cell and hemochrome in the blood, and the speed up of the synthesis of both albumin and globulin.

Also, laser irradiation on the specific receptor sites stimulates T cells and beta cells in the body rendering an increase of their glycogen contents as well as an increase of the activity of ribonucleic acid.

Furthermore, it is observed that the secretion of plasma cortisol, adrenaline, noradrenaline and dopamine of a light smoker can be regulated to a normal condition after the laser treatment.

All these biochemical effects to the body brings the disease and medical conditions associated with smoking to a shift towards physiological steadiness. It also gradually removes the toxic substances accumulated inside the body and at the same time increases the resistance to the toxic substances remained inside the body such that the body is better equipped against diseases.

Another point needed to be stressed here is that laser can be considered as electromagnetic waves of a particular wave length and that a human body as an electromagnetic field. Although laser irradiated only at certain receptor sites on the body of a patient, it generates a chain reaction on the electromagnetic field and hence produces all-round effects to the body. Thus the laser treatment hereinbefore disclosed not only help patient quit smoking, but also activates the cells, speeds up the metabolic rate, and regulates the physiological functions of the body of the patient.

The method in accordance with the present invention works particular well on heavy smokers who often find that they suffer from a variety of physical and mental side-effects when they give up smoking.

While the present invention has been shown and described with reference to a particular example, it should be noted that various other changes or modifications may be made without departing from the scope of the present invention.

## Claims

1. A method of treatment to stop smoking comprising:

(a) a first step of applying a bioelectrical-stimulator to stimulate the body of a subject via surface electrodes;

(b) a second step, simultaneously carried out with said first step, of applying lasers bilaterally to irradiate a first pair of auricular points on the auricles of the subject;

(c) a third step, simultaneously carried out with said first step, of applying lasers bilaterally to irradiate a second pair of auricular points on the auricles of the subject;

(d) a fourth step, simultaneously carried out with said firts step, of applying lasers bilaterally to irradiate a third pair of auricular points on the auricles of the subject;

(e) a fifth step of applying small magnetic discs to said first, second and third pair of auricular points; and

(f) a sixth step of repeating said first, second, third, fourth and fifth steps to effect gradual reduction of smoking until the point of total abstinence is reached.

2. A method as claimed in claim 1, wherein said lasers have an output level of five milliwatts.

3. A method as claimed in claim 1, wherein said first step lasts for at least fifteen minutes.

4. A method as claimed in claim 1, wherein said second step lasts for at least five minutes.

5. A method as claimed in claim 1, wherein said third step lasts for at least five minutes.

6. A method as claimed in claim 1, wherein said fourth step lasts for at least five minutes.

7. A method as claimed in claim 2, wherein said magnetic discs have a magnetic strength of 2500 GAUS.

8. A method as claimed in claim 1, wherein said first pair of auricular points is Spirit Door.

9. A method as claimed in claim 1, wherein said second pair of auricular points is Esophagus.

10. A method as claimed in claim 1, wherein said third pair of auricular points is Lung.

11. A method as claimed in claim 1, wherein said electrical stimulation is applied to HEART 7 and SPLEEN 6 on the subject.

12. A method substantially as hereinbefore described with reference to the accompanying drawings.

13. Apparatus for treatment to stop smoking comprising at least two laser apparatus adapted to be operable to produce output beams of five milliwatts and to irradiate certain auricular points on the auricles of a subject.

14. Apparatus as claimed in claim 13 in combination with electrotherapeutic apparatus and magnetotherapeutic apparatus.

15. Apparatus as claimed in claim 14, wherein said electrotherapeutic apparatus is a bioelectrical-stimulator adapted to generate electrical stimulation through surface electrodes.

16. Apparatus as claimed in claim 14, wherein said magnetotherapeutic apparatus takes the form of small magnetic discs.

17. Apparatus as claimed in claim 13, wherein said laser is a Helium-Neon laser.

18. Apparatus as claimed in claim 15, wherein said surface electrodes are of metal.

19. Apparatus as claimed in claim 15, wherein said surface electrodes are of conductive plastic material.

20. Apparatus as claimed in claim 16, wherein said magnetic discs have magnetic strength of 2500 GAUS.

21. Apparatus substantially as hereinbefore described with reference to Figure 1 of the accompanying drawings.

FIG. 1

FIG. 2

FIG. 3

```
┌─────────────────────────────┐        ┌─────────────────────────────┐
│ applying bioelectrical-     │        │ applying lasers with output │
│ stimulator to stimulate     │        │ of 5 mw bilaterally to      │
│ HEART 7 and SPLEEN 6        │        │ irradiate a first pair of   │
│ of the subject's body via   │        │ auricular points on the     │
│ surface electrodes          │        │ subject's auricles for at   │
└─────────────────────────────┘        │ least 5 minutes             │
                                       └─────────────────────────────┘

                                       ┌─────────────────────────────┐
                                       │ applying lasers with output │
                                       │ of 5 mw bilaterally to      │
                                       │ irradiate a second pair of  │
                                       │ auricular points on the     │
                                       │ subject's auricles for at   │
                                       │ least 5 minutes             │
                                       └─────────────────────────────┘

                                       ┌─────────────────────────────┐
                                       │ applying lasers with output │
                                       │ of 5 mw bilaterally to      │
                                       │ irradiate a third pair of   │
                                       │ auricular points on the     │
                                       │ subject's auricles for at   │
                                       │ least 5 minutes             │
                                       └─────────────────────────────┘

        ┌─────────────────────────────────────┐
        │ applying small magnetic discs of    │
        │ magnetic strength of 2500 GAUS to   │
        │ the three pairs of auricular points │
        └─────────────────────────────────────┘

        ┌─────────────────────────────────────┐
        │ repeating the laser, electro-       │
        │ therapeutic and magnetotherapeutic  │
        │ treatments to effect gradual        │
        │ reduction of smoking until          │
        │ subject totally stops smoking       │
        └─────────────────────────────────────┘
```

*FIG. 4*

European Patent Office

**PARTIAL EUROPEAN SEARCH REPORT**
which under Rule 45 of the European Patent Convention
shall be considered, for the purposes of subsequent
proceedings, as the European search report

Application number

EP 87 30 7906

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A - 0 109 935 (SYMTONIC S.A.)<br><br>* Page 3, line 25 - page 6, line 15; page 9, line 28 - page 10, line 3; page 12, lines 1-35; figures 1-3,7b * | 1-18 | A 61 N 5/06<br>A 61 N 1/32<br>A 61 N 1/42 |
| A | FR - A - 1 589 067 (BERNAZ)<br><br>* Page 2, line 16 - page 7, line 19; figures 1-7 * | 1-6, 8-15, 17-19 | |
| A | EP - A - 0 100 050 (BLUM)<br><br>* Page 9, line 16 - page 11, line 1; figures 1,2 * | 1,6, 7,14, 16,20 | |
| A | GB - A - 2 095 118 (SKOVAJSA)<br><br>* Page 1, lines 52-114; figures 1-4 * | 1,6, 7,14, 16,20, 21 | TECHNICAL FIELDS SEARCHED (Int. Cl.4)<br><br>A 61 N<br>A 61 H |

-2-

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 13-21

Claims searched incompletely: 1-12

Claims not searched:

Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 12-04-1988 | SCHMIERER |

| Category | Citation of document with indication, where appropriate, of relevant passages | Relevant to claim | CLASSIFICATION OF THE APPLICATION (Int. Cl 4) |
|---|---|---|---|
| | **DOCUMENTS CONSIDERED TO BE RELEVANT** | | |
| A | EP - A - 0 160 753 (INT. MEDICAL MACHINES) <br><br> * Page 5, line 11 - page 8, line 17; figures 1-3 * <br> -- | 1,14, 15,19 | |
| A | ACUPUNCTURE & ELECTRO-THERAPEUTICS RESEARCH, INT.J, vol. 8, no.3-4,1983 pages 297-302 Pergamon Press Ltd. US; V.N. ZALESSKIY et al.: "Laser-acupuncture reduces cigarette smoking: a preliminary report" <br><br> -------------------- | | |

TECHNICAL FIELDS SEARCHED (Int. Cl.4)